# EUROPEAN PATENT APPLICATION

(11) **EP 4 083 625 A1**
(43) Date of publication of application: **02.11.2022**
(21) Application number: 20905866.8
(22) Date of filing: 24.12.2020
(51) Int. Cl.: G01N 33/543

(54) **IMMUNOCHROMATOGRAPHIC STRIP, IMMUNOCHROMATOGRAPHIC DEVICE, IMMUNOCHROMATOGRAPHIC KIT, AND METHOD FOR DETECTING TEST SUBSTANCE**

(30) Priority: 25.12.2019 JP 2019234034
(71) Applicant: Fujirebio Inc., Shinjuku-ku Tokyo 163-0410 (JP)
(72) Inventor: HIROSE Ryo, Tokyo 163-0410 (JP); ISHIKAWA Naoki, Hachioji-shi, Tokyo 192-0031 (JP); YOSHINAGA Takaharu, Tokyo 163-0410 (JP); NIWA Toshihiro, Tokyo 163-0410 (JP)
(74) Representative: Müller-Boré & Partner Patentanwälte PartG mbB
(86) International application number: PCT/JP2020/048473
(87) International publication number: WO 2021/132470

(57) **Abstract**

An immunochromatographic strip for use in detecting a test substance in a sample by immunochromatography, comprising: a labeled body-containing section containing a blocked labeled antibody in which a labeling substance and an antibody capable of binding to a test substance are immobilized on a water-soluble carrier made of a water-soluble polymer.

## Description

### [Technical Field]

The present invention relates to an immunochromatographic strip, an immunochromatographic device, an immunochromatographic kit, and a method for detecting test substance, and more specifically, to an immunochromatographic strip, an immunochromatographic device comprising the same, an immunochromatographic kit comprising at least one of these, and a method for detecting test substance using at least one of these.

### [Background Art]

In order to determine positive or negative for an infectious disease and specify the cause of the infectious disease, it is necessary to detect a pathogen such as a bacterium or a virus in a sample collected from a subject. In addition, in order to detect positive or negative for a specific disease, it is also known to detect a substance that serves as a marker for the disease in a sample such as blood. As a method for detecting a test substance such as a pathogen or a marker, immunoassay methods, which utilize antigen-antibody reactions, are known.

As the immunoassay methods, for example, methods in which after a test substance is captured by an antibody immobilized on a solid phase such as ELISA, the antibody labeled with an enzyme is subjected to secondary reaction to measure the enzyme activity are known, and many studies have conventionally been conducted. For example, International Publication No. WO2006/070732 (PTL 1) states the use of a blocked enzyme probe complex in which a carrier having a specific molecular weight is bound via an enzyme, and a probe molecule is bound to the complex in which the enzyme is bound to the carrier for the purpose of improving detection sensitivity by these methods.

On the other hand, among the immunoassay methods, immunochromatography method is known as a simple and rapid detection method. The immunochromatography method includes, for example, a method including: developing a sample from one end of an immunochromatographic strip (test piece) made of a porous membrane on which an antibody capable of binding to a test substance in the sample is immobilized; forming a complex of a labeled body capable of binding specifically to the test substance (a substance capable of binding specifically to the test substance and is labeled (a labeled antibody or the like)) and the test substance; and capturing the complex indirectly or directly using an antigen, an antibody, or the like immobilized on the above porous membrane to detect the test substance. Since such a method can detect the test substance in the sample only by applying or dropping the sample onto the immunochromatographic strip and developing the sample, the complex, or the like on the porous membrane under capillary action, the operation is easy. In addition, since such a method is capable of detection of a test substance in a short period of time and also allows visual judgments, the method is widely utilized in research experiments and clinical tests.

As such an immunochromatographic technique, for example, Japanese Unexamined Patent Application Publication No. 2015-1395 (PTL 2) describes the use of a developer for immunochromatography that contains a water-soluble polysaccharide having a specific viscosity and contains a detection reagent labeled with a carrier having an average particle size of 1 nm or more to 60 nm or less for the purpose of improving the detection sensitivity by such a method. In addition, "ESPLINE (registered trademark)" in which alkaline phosphatase (ALP)-labeled antibodies are set on membranes is manufactured and sold by FUJIREBIO Inc. as reagents for detecting influenza virus antigens, HIV antigens, and the like.

### [Citation List]

### [Patent Literature]

[PTL 1] International Publication No. WO2006/070732
[PTL 2] Japanese Unexamined Patent Application Publication No. 2015-1395

### [Summary of Invention]

### [Technical Problem]

When further studied method for detecting a test substance in a sample by immunochromatography, the present inventors have found that the conventional detection methods using the labeled antibodies such as ALP labeled antibodies have not been sufficient yet in terms of the detection sensitivities, and in a case where the concentration of the test substance in the sample is low, the test substance cannot be detected, that is, the signal intensity to be detected is weak (leading to a negative result) in some cases. In addition, in this case, although it is possible to enhance the signal intensity to be detected by increasing the concentration of the labeling substance or the concentration of the antibody, since this also enhances background signal intensity, it is difficult to enhance the detection sensitivity.

The present invention has been made in view of the above-described problem, and an object thereof is to provide an immunochromatographic strip that is capable of detection of a test substance with high sensitivity even when the concentration of the test substance is low, an immunochromatographic device comprising the same, an immunochromatographic kit comprising at least one of these, and a method for detecting test substance that is capable of detection of a test substance with high sensitivity even when the concentration of the test substance is low by immunochromatography.

### [Solution to Problem]

The present inventors conducted earnest studies in order to achieve the above object, and consequently have found that it enables detection of a test substance with high sensitivity without enhancing background signal intensity even when the concentration of the test substance in a sample is low by using a blocked labeled antibody in which a labeling substance and an antibody capable of binding to a test substance are immobilized on a water-soluble carrier made of a water-soluble polymer, as a labeled body to be contained in a strip for use in detecting the test substance in the sample by immunochromatography, and have led to the completion of the present invention.

That is, the present invention relates to an immunochromatographic strip, an immunochromatographic device comprising the same, an immunochromatographic kit comprising these, and a method for detecting test substance using these, and more specifically provides the following:
[1] An immunochromatographic strip for use in detecting a test substance in a sample by immunochromatography, comprising:
   a labeled body-containing section containing a blocked labeled antibody in which a labeling substance and an antibody capable of binding to a test substance are immobilized on a water-soluble carrier made of a water-soluble polymer.
[2] The immunochromatographic strip according to [1], wherein
   an average particle size of the blocked labeled antibody is 50 nm or more.
[3] The immunochromatographic strip according to [1] or [2], wherein
   the water-soluble polymer is at least one selected from the group consisting of sugars and amino acids.
[4] The immunochromatographic strip according to any one of [1] to [3], further comprising:
   a capturing body-immobilized section on which a capturing body capable of capturing a test substance bound to the blocked labeled antibody is immobilized.
[5] An immunochromatographic device for use in detecting a test substance in a sample by immunochromatography, comprising:
   the immunochromatographic strip according to any one of [1] to [4].
[6] An immunochromatographic kit for use in detecting a test substance in a sample by immunochromatography, comprising:
   at least one selected from the group consisting of the immunochromatographic strip according to any one of [1] to [4] and the immunochromatographic device according to [5] .
[7] A method for detecting test substance for detecting a test substance in a sample by immunochromatography, comprising:
   in an immunochromatographic strip comprising a labeled body-containing section containing a blocked labeled antibody in which a labeling substance and an antibody capable of binding to a test substance are immobilized on a water-soluble carrier made of a water-soluble polymer,
   a labeling step of bringing a sample and the blocked labeled antibody into contact to form a complex of a test substance in the sample and the blocked labeled antibody;
   a capturing step of capturing the complex; and
   a detecting step of detecting the captured complex.
[8] The method for detecting test substance according to [7], wherein
   an average particle size of the blocked labeled antibody is 50 nm or more.
[9] The method for detecting test substance according to [7] or [8], wherein
   the water-soluble polymer is at least one selected from the group consisting of sugars and amino acids.
[10] The method for detecting test substance according to any one of [7] to [9], wherein
   the immunochromatographic strip further comprises a capturing body-immobilized section on which a capturing body capable of capturing a test substance bound to the blocked labeled antibody is immobilized, and
   the capturing step is a step of capturing the complex by means of a test substance bound to the blocked labeled antibody in the capturing body-immobilized section.

Note that although the reason why the above-described object is achieved by the configuration of the present invention, the present inventors surmise as follows. That is, since in the blocked labeled antibody to be contained in the labeled body-containing section of the immunochromatographic strip of the present invention, since the labeling substance and the antibody capable of binding to a test substance are immobilized on the water-soluble carrier made of a water-soluble polymer, a complex in which pluralities of labeling substances and antibodies are in series by the water-soluble carrier, which is a polymer, is formed. Hence, for example, in the case where this forms an immune complex with a test substance and the antibody and is captured in the capturing body-immobilized section of the immunochromatographic strip, the amount of the labeling substance to be captured in the capturing body-immobilized section consequently becomes larger than the case of using a labeled antibody composed of only a labeling substance and an antibody. The present inventors surmise that since in immunochromatography, the detection of a test substance is conducted by confirming a detection of an/the test substance derived from a labeling substance to be captured in the capturing body-immobilized section, it is possible to sufficiently increase the signal intensity and making the detection sensitivity higher by using the immunochromatographic strip of the present invention, which is capable of making the amount of the labeling substance to be captured in the capturing body-immobilized section larger than the conventional techniques, as described above.

### [Advantageous Effects of Invention]

The present invention makes it possible to provide an immunochromatographic strip that is capable of detection of a test substance with high sensitivity even when the concentration of the test substance is low, an immunochromatographic device comprising the same, and an immunochromatographic kit comprising at least one of these, and a method for detecting test substance that is capable of detection of a test substance with high sensitivity by immunochromatography even when the concentration of the test substance is low.

### [Brief Description of Drawings]

[FIG. 1] FIG. 1 is a schematic plan view showing one embodiment of an immunochromatographic strip of the present invention.
[FIG. 2] FIG. 2 is a schematic cross-sectional view showing the one embodiment of the immunochromatographic strip of the present invention.
[FIG. 3] FIG. 3 is a schematic cross-sectional view showing one embodiment of a main part of an immunochromatographic device of the present invention.
[FIG. 4] FIG. 4 is a schematic plan view showing the one embodiment of the immunochromatographic device of the present invention.
[FIG. 5] FIG. 5 is a schematic cross-sectional view showing the one embodiment of the immunochromatographic device of the present invention.

### [Description of Embodiments]

Hereinafter, the present invention will be described in detail based on preferred embodiments thereof.

An immunochromatographic strip of the present invention is a strip for use in detecting a test substance in a sample by immunochromatography, and comprises a labeled body-containing section containing a blocked labeled antibody in which a labeling substance and an antibody capable of binding to a test substance are immobilized on a water-soluble carrier composed of a water-soluble polymer.

### (Test substance (Analyte))

In the present invention, the "test substance" to be detected is not particularly limited, and includes pathogens such as bacteria, protists, fungi, viruses and substances such as sugar chains derived from these; and substances used as markers for specific diseases, and more specifically includes the pathogens, proteins, polysaccharides, nucleic acids, and the like. The pathogens include, for example, HIV (human immunodeficiency virus), influenza viruses (type A, type B, and the like), Zika virus, HBV (hepatitis B virus), and the like, but are not limited to these.

### (Sample)

The "sample" used for detection of a test substance in the present invention is not particularly limited as long as the test substance can exist in the sample. The sample includes, for example, samples such as the whole blood, the serum, the plasma, the urine, the feces, and the mucoid secretion (the induced sputum, the pharyngeal swab, the nasal swab, the saliva, and the otorrhea) collected from a subject (for example, a mammal, preferably a human), but is not limited to these. The sample is preferably a watery sample, and more preferably an aqueous solution or a water dispersion liquid, or may be what is obtained by diluting the sample with a diluent, treating the sample with various pretreatment liquids, or filtering the sample, and the like. The diluent includes, for example, a buffer solution, and the buffer solution includes a buffer solution adjustable to pH favorable for the target antigen-antibody reaction, and more specifically, a phosphate buffer solution, a Tris buffer solution, a Good's buffer solution, a boric acid buffer solution, and the like. The pretreatment liquid includes, for example, a surfactant.

### (Detection)

In the present invention, the "detection of a test substance" includes detection to check the presence or absence of the test substance, and quantification or semi-quantification of the amount of the test substance. In the present invention, the detection of a test substance is conducted by detecting a signal derived from the labeling substance and quantifying the intensity of the signal as necessary, and whether or not the detection has been successful is determined by detecting a signal in a development confirmation zone described below as necessary, and the above "signal" includes color reaction (color production), reflected light, luminescence, fluorescence, radiation by radioisotope, and the like, and also includes what can be observed by a detection method·device depending on the type of the signal besides what can be observed by the naked eye.

### (Blocked Labeled Antibody)

In the present invention, the "blocked labeled antibody" is a complex that includes a water-soluble carrier made of water-soluble polymer, a labeling substance, and an antibody capable of binding to the above test substance, in which the labeling substance and the antibody are immobilized on the water-soluble carrier, and is a conjugate in which the labeling substance, the antibody, and the water-soluble carrier are directly or indirectly bound together. The blocked labeled antibody of the present invention may be such that a plurality of labeling substances and/or a plurality of antibodies may be bound to one molecule of the water-soluble carrier.

The blocked labeled antibody of the present invention only has to be such that the labeling substance and the antibody are carried by the water-soluble carrier, and may be such that the labeling substance and the antibody are bound to the water-soluble carrier independently from each other, or may be such that any of the water-soluble carrier, the labeling substance, and the antibody is bound to the other two, or may be such that one of the antibody or the labeling substance is bound to the water-soluble carrier via the other of the labeling substance or the antibody.

### [Water-soluble Carrier]

The water-soluble carrier contained in the blocked labeled antibody of the present invention functions as a carrier that carries mainly the labeling substance and the antibody and is made of a water-soluble polymer. The water-soluble polymer making the water-soluble carrier according to the present invention is not particularly limited as long as the water-soluble polymer can fix and carry the labeling substance and the antibody. In the present invention, the "water-soluble polymer" means a polymer whose solubility to water at room temperature under ordinary pressure exceeds 0.01 g/mL, preferably is 0.05 g/mL or more, and more preferably is 0.1 g/mL or more .

The weight-average molecular weight of the water-soluble polymer according to the present invention (a weight-average molecular weight measured with a molecular weight marker in gel filtration chromatography (GFC), the same applies hereinafter) is preferably 30,000 to 1,000,000 from the viewpoint of the sensitivity of the detection and the viewpoint of being water soluble, more preferably 50,000 to 500,000, and further preferably 70,000 to 250,000, from the viewpoint that it tends to allow a blocked labeled antibody having a more preferable average particle size to be obtained.

In addition, the average mass (an average mass measured with a marker in gel filtration chromatography (GFC), the same applies hereinafter) of the water-soluble polymer according to the present invention is preferably 50, 000 to 500,000 Da, and more preferably 70,000 to 250,000 Da, from the viewpoint that it tends to allow a blocked labeled antibody having a more preferable average particle size to be obtained.

The water-soluble polymer according to the present invention includes, for example, sugars such as dextran, amino dextran, Ficoll (trade name), dextrin, agarose, pullulan, various celluloses (for example, hemicellulose and lignin), chitin, chitosan, and modification products of these (for example, hydrazine dextran) ; proteins such as β-galactosidase and thyroglobulin; polypeptides; DNAs; hemocyanins; amino acids such as polylysine, and one of these may be used alone or two or more of these may be used in combination. Among these, the water-soluble polymer according to the present invention is preferably at least one selected from the group consisting of sugars and amino acids, and more preferably at least one selected from the group consisting of sugars such as dextran, amino dextran, Ficoll (trade name), dextrin, agarose, pullulan, hydrophilic cellulose, and modification products of these; and amino acids such as polylysine, from the viewpoint that they have lower immunogenicity, lower ionicity, and are more hydrophilic. Moreover, the water-soluble polymer according to the present invention is further preferably dextran from the viewpoint that a blocked labeled antibody can be more easily produced, the average particle size of the blocked labeled antibody can be more easily controlled, a failure in development of the blocked labeled antibody is less likely to occur, the measurement sensitivity can be more uniformly maintained because dextran can be easily restored from a dried state during development, and the like.

### [Labeling Substance]

The labeling substance contained in the blocked labeled antibody of the present invention mainly functions as a label of the blocked labeled antibody, and those used as labeling substances in the publicly-known immunoassay methods may be used with no particular limitation.

The labeling substance according to the present invention includes, for example, enzymes; radioisotopes; luminescent substances such as acridinium derivatives; fluorescent substances such as europium; fluorescent proteins such as allophycocyanin (APC) and phycoerythrin (R-PE); low molecular weight labeling substances such as fluorescein isothiocyanate (FITC) and rhodamine isothiocyanate (RITC); gold particles; avidin; biotin; latex; dinitrophenyl (DNP) ; and digoxigenin (DIG), and one of these may be used alone or two or more of these may be used in combination. Among these, the labeling substance according to the present invention is preferably an enzyme from the viewpoint that a failure in development of the blocked labeled antibody is less likely to occur, and visual detection is easier. In the case where an enzyme is used as the labeling substance, it is possible to conduct various detections depending on the substrate by using a chromogenic substrate, a fluorescent substrate, a chemiluminescent substrate, or the like as the substrate.

The enzyme includes various enzymes that have conventionally used in the enzyme immunoassay methods, and includes, for example, horseradish peroxidase (HRP), alkaline phosphatase (ALP), β-galactosidase (β-gal), glucose oxidase, and luciferase, but is not limited to these. In addition, the radioisotope includes, for example, isotopes of iodine, tritium, carbon, and the like, and can label using a method that uses a Bolton-Hunter reagent or the like, for example.

### [Antibody]

The "antibody capable of binding to a test substance" contained in the blocked labeled antibody of the present invention is an antibody that is capable of binding to the test substance and capable of capturing the test substance. The antibody may be a polyclonal antibody or a monoclonal antibody. In addition, in the present invention, the "antibody" includes not only complete antibodies but also antibody fragments (for example, Fab, Fab', F(ab')₂, Fv, single-chain antibodies, diabodies, and the like) and low molecular weight antibodies in which a variable region of an antibody is bound. Among these, the antibody contained in the blocked labeled antibody of the present invention is preferably an antibody fragment from the viewpoint that a failure in development of the blocked labeled antibody is less likely to occur, the average particle size of the blocked labeled antibody can be more easily controlled.

The antibody can be produced by employing and modifying a conventionally publicly-known production method as appropriate depending on the type of the test substance and the like, and an antibody distributed on the market in general may be used as appropriate.

### [Configuration of Blocked Labeled Antibody]

In the blocked labeled antibody of the present invention, the content of the labeling substance is not particularly limited but is preferably set such that the number of molecules of the labeling substance binding to one molecule of the water-soluble polymer becomes as large as possible in order to further improve the detection sensitivity. For example, in the case where the labeling substance is an enzyme, the mass of the labeling substance (in the case where the labeling substance is a combination of two or more, the total mass of these) to the 100 parts by mass of the water-soluble polymer (in the case where the water-soluble polymer is a combination of two or more, the total mass of these, the same applies hereinafter) is preferably 200 to 1,000 parts by mass, and more preferably 300 to 800 parts by mass.

In the blocked labeled antibody of the present invention, the content of the antibody is not particularly limited but is preferably set such that the number of molecules of the antibody binding to one molecule of the water-soluble polymer becomes as large as possible in order to further improve the detection sensitivity. For example, the mass of the antibody (in the case where the antibody is a combination of two or more, the total mass of these) to 100 parts by mass of the water-soluble polymer is preferably 400 to 20,000 parts by mass, and more preferably 500 to 1,500 parts by mass.

In addition, the weight-average molecular weight of the blocked labeled antibody of the present invention is preferably 1,000,000 to 3,000,000, and more preferably 1,500,000 to 2,600,000, per molecule of the blocked labeled antibody. If the weight-average molecular weight is less than the lower limit, the detection sensitivity tends to decrease. On the other hand, if the weight-average molecular weight is more than the upper limit, although the detection sensitivity is improved, aggregation and the like tends to be likely to occur in the aqueous solution.

In addition, as the blocked labeled antibody of the present invention, the average particle size of a complex exclusive of the antibody, that is, a complex in which the labeling substance is immobilized on the water-soluble carrier (hereinafter, sometimes referred to as the "label carrier") is preferably 35 nm or more, more preferably 40 to 185 nm, further preferably 50 to 135 nm, and particularly preferably 60 to 125 nm. If the average particle size of the label carrier is less than the lower limit, the detection sensitivity tends to decrease. On the other hand, if the average particle size of the label carrier is more than the upper limit, a failure in development of the blocked labeled antibody tends to be likely to occur.

In addition, in the case where the antibody is an antibody fragment (preferably, having a longest diameter of 15 nm or more), for example, the average particle size of the blocked labeled antibody of the present invention is preferably 50 nm or more, more preferably 55 to 200 nm, further preferably 65 to 150 nm, and particularly preferably 75 to 140 nm. If the average particle size of the blocked labeled antibody is less than the lower limit, the detection sensitivity tends to decrease. On the other hand, if the average particle size of the blocked labeled antibody is more than the upper limit, a failure in development of the blocked labeled antibody tends to be likely to occur.

The average particle sizes of the label carrier and the blocked labeled antibody are each an average particle size obtained by measuring and analyzing a label carrier solution or a blocked labeled antibody solution whose concentration in a buffer (a 0.1M phosphoric acid buffer (pH 7.0) or a 0.1M Tris-HCl buffer (pH 8.0)) is 0.07 to 0.8 mg/mL using a Zeta potential·particle size-molecular weight measurement system (ELSZ-2000ZS, manufactured by OTSUKA ELECTRONICS Co., LTD.) under conditions set in the system by default. The detail of the specific conditions for the measurement and analysis are as described in Examples below. Note that in the present invention, the particle sizes of the label carrier and the blocked labeled antibody each include the particle sizes of primary particles and secondary particles (aggregate) formed by aggregation of these primary particles.

The average particle sizes of the label carrier and the blocked labeled antibody can be adjusted, for example, by using a water-soluble polymer having an appropriate molecular weight or mass as the water-soluble polymer, by modifying the water-soluble polymer, or in the case where the water-soluble polymer is dextran, for example, by adjusting the oxidation conditions (the concentration of the oxidant, the oxidation time, and the like), or the like.

### [Method for Producing the Blocked Labeled Antibody]

The blocked labeled antibody of the present invention can be produced by immobilizing the labeling substance and the antibody on the water-soluble carrier. As such a production method, a conventionally publicly-known method or a method based on this may be employed, and the labeling substance and the antibody (hereinafter, sometimes referred to collectively as the "carried substances") may be directly immobilized or indirectly immobilized on the water-soluble carrier.

The method for directly immobilizing the carried substances on the water-soluble carrier includes, for example, a method that adds an active group such as a carboxy group, an epoxy group, a tosyl group, an amino group, a hydroxy group, an isothiocyanate group, an isocyanate group, an azide group, an aldehyde group, a carbonate group, an allyl group, an aminooxy group, a maleimide group, or a thiol group to the carried substances and/or the water-soluble polymer or uses a substance having the active group as the carried substances and/or the water-soluble carrier to immobilize the carried substances on the water-soluble carrier through covalent bonding of the active group and the carried substances and/or the water-soluble polymer. As the carried substances and the water-soluble polymer to which the active group is added, commercially-available ones may be used as they are, or these may be prepared by introducing the active group into the surfaces of the carried substances and the water-soluble polymer under appropriate reaction conditions. As examples, a thiol group may be introduced by, for example, using a commercially-available reagent such as S-acetyl mercaptosuccinic anhydride, N-succinimidyl 3-(2-pyridyldithio)propionate, or 2-iminothiolane hydrochloride. A maleimide group may be introduced into an amino group by, for example, using a commercially-available reagent such as N-(6-maleimidocaproyloxy)succinimide or N-(4-maleimidobutyryloxy)succinimide. A pyridyl disulfide group may be introduced by, for example, using a commercially-available reagent such as N-Succinimidyl 3-(2-pyridyldithio)propionate (SPDP), N-{6-[3-(2-Pyridyldithio)propionamido]hexanoyloxy}sulf osuccinimide, or sodium salt (Sulfo-AC5-SPDP).

In addition, the method for indirectly immobilizing the carried substances on the water-soluble carrier includes, for example, a method for indirectly immobilizing the carried substances on the water-soluble carrier by means of a linker such as polyhistidine, polyethylene glycol, oligopeptides containing cysteine and/or lysine, linker molecules having the active groups (for example, hydrazine salt, hydrazide, AMAS (N-α-maleimidoacet-oxysuccinimide ester), BMPS (N-β-maleimidopropyl-oxysuccinimide ester), GMBS (N-γ-maleimidobutyryl-oxysuccinimide ester), MBS (m-maleimidobenzoyl-N-hydroxysuccinimide ester), SMCC (succinimidyl 4-(N-maleimidomethyl)eyelohexane-1-carboxylate), E MCS (N-ε-malemidocaproyl-oxysuccinimide ester), SMPB (succinimidyl 4-(p-maleimidophenyl)butyrate), SMPH (Succinimidyl 6-((beta-maleimidopropionamido)hexanoate)), LC-SMCC (succinimidyl 4-(N-maleimidomethyl)cyclohexane-1-carboxy-(6-amidocap roate)), Sulfo-KMUS (N-κ-maleimidoundecanoyl-oxysulfosuccinimide ester), SIA (succinimidyl iodoacetate), SBAP (succinimidyl 3-(bromoacetamido)propionate), SIAB (succinimidyl (4-iodoacetyl)aminobenzoate), Sulfo-SANPAH (sulfosuccinimidyl 6-(4'-azido-2'-nitrophenylamino)hexanoate), SDA (NHS-Diazirine)(succinimidyl 4,4'-azipentanoate), Sulfo-SDAD (Sulfo-NHS-SS-Diazirine)(sulfosuccinimidyl 2-((4,4'-azipentanamido)ethyl)-1,3'-dithiopropionate), EDC (1-ethyl-3-(3-dimethylaminopropyl)carbodiimide hydrochloride), NHS (N-hydroxysuccinimide), BMPH (N-β-maleimidopropionic acid hydrazide), EM CH (N-ε-maleimidocaproic acid hydrazide), MPBH (4-(4-N-maleimidophenyl)butyric acid hydrazide), KMUH (N-κ-maleimidoundecanoic acid hydrazide), PDPH (3-(2-pyridyldithio)propionyl hydrazide), PMPI (p-maleimidophenyl isocyanate), and SPB (succinimidyl-[4-(psoralen-8-yloxy)]-butyrate)), and the like. The selection and size of the linker may be set as appropriate in consideration of the strength of the bond with the carried substances and/or the water-soluble polymer, steric hindrance due to the fixation of the carried substances to the water-soluble carrier, and the like.

In the method for producing a blocked labeled antibody of the present invention, the labeling substance and the antibody may be immobilized on the water-soluble carrier together or separately in series. However, it is preferable to first immobilize one of them (more preferably, the labeling substance) on the water-soluble carrier and then immobilize the other (more preferably, the antibody) thereto from the viewpoint of the easiness of production and easiness of controlling the amount of the labeling substance and the amount of the antibody.

Such a production method is not particularly limited and for example, in the case where the labeling substance is an enzyme, the water-soluble polymer is first oxidized with an oxidant such as sodium periodate (NaIO₄) to be added with an aldehyde group, is then reacted with hydrazine hydrochloride, and thereafter, this is reacted with a reducing agent such as dimethylamine-borane (DMAB) into a hydrazine as described in Examples below. On the other hand, the enzyme is also oxidized with an oxidant such as sodium periodate to be added with an aldehyde group. Subsequently, the aldehyde group and the hydrazine residue added to the water-soluble polymer as described above are reacted with each other to form a hydrazone bond to obtain a water-soluble polymer-enzyme conjugate (label carrier) . Subsequently, the water-soluble polymer-enzyme conjugate thus obtained is treated with a crosslinker (for example, sulfo-E MCS or the like) to introduce a maleimide group. In addition, a thiol group is added to the antibody with a thiolating reagent (thiolation) and is bound with the maleimide group introduced into the water-soluble polymer-enzyme conjugate, so that the three of the water-soluble polymer (water-soluble carrier)-enzyme-antibody can be covalent bonded. The ratio of the water-soluble polymer, the labeling substance, and the antibody supplied for these reactions may be selected as appropriate so as to achieve the preferable ranges of the respective contents in the above-described blocked labeled antibody.

### (Immunochromatographic strip, Immunochromatographic device)

The immunochromatography method is an immunoassay method that develops a sample from one end or one point of a strip (test piece) and detects a test substance through an antigen-antibody reaction in the course of the development. The immunochromatographic strip of the present invention comprises a labeled body-containing section containing the blocked labeled antibody. In addition, the immunochromatographic device of the present invention comprises the immunochromatographic strip of the present invention.

Hereinafter, preferred embodiments of the immunochromatographic strip and the immunochromatographic device of the present invention will be described in detail by giving examples with reference to the drawings. However, the present invention is not limited to these. Note that the same or corresponding elements are denoted with the same reference signs and duplicate descriptions are omitted in the description and drawings below.

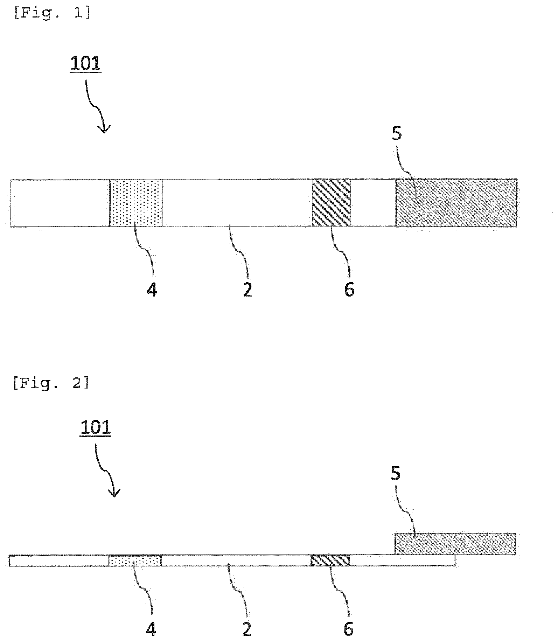

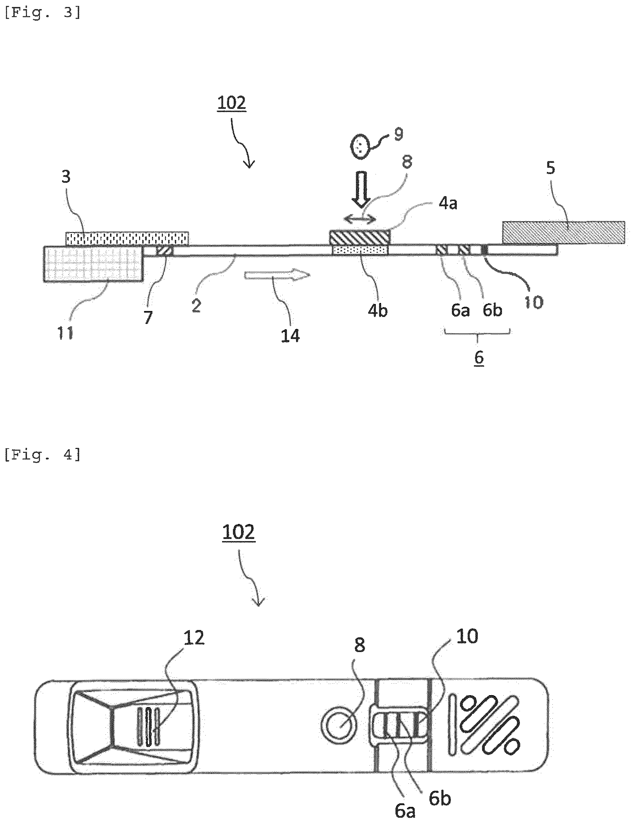

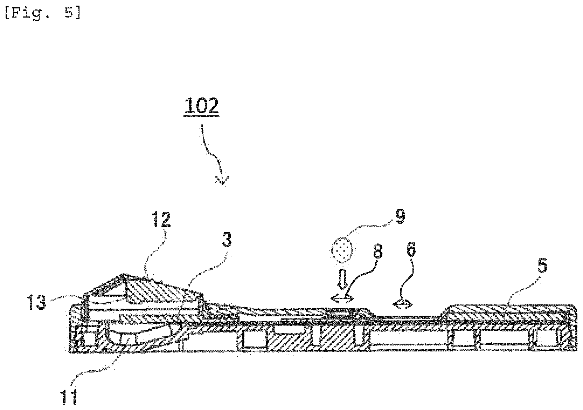

FIG. 1 is a schematic plan view of one embodiment (an immunochromatographic strip 101) of the immunochromatographic strip of the present invention, and FIG. 2 is a cross-sectional view of the same. In addition, FIG. 3 is a schematic cross-sectional view showing one embodiment (an immunochromatographic device 102) of a main part of the immunochromatographic device of the present invention. Moreover, FIG. 4 is a schematic plan view showing one embodiment of the immunochromatographic device of the present invention, and FIG. 5 is a cross-sectional view of the same. In FIGs. 1 to 5, the immunochromatographic strip is an immunochromatographic strip of lateral flow type. In the following description, the absorbent zone 5 side of a matrix 2 is referred to as downstream (that is, the downstream side in the direction of development of the sample (a direction of development 14 in FIG. 3)) while the opposite side is referred to as upstream (that is, the upstream side in the direction of development of the sample (the direction of development 14 in FIG. 3)).

### [Matrix]

The immunochromatographic strip of the present invention comprises: a labeled body-containing section (for example, a labeled body-containing section 4) containing at least the blocked labeled antibody; and a matrix (for example, a matrix 2) having the labeled body-containing section 4.

The matrix according to the present invention is an insoluble carrier that functions as an immunochromatographic medium (an immobile phase). In addition, the matrix is also an antibody-containing membrane having a labeled body-containing section, and a capturing body-immobilized section, a development confirmation zone, and the like as necessary as a main body of the immunochromatographic strip.

The matrix according to the present invention is preferably a porous membrane, and includes, for example, a nitrocellulose membrane, a nitrocellulose-mixed ester membrane, a cellulose membrane, an acetylcellulose membrane, a polysulfone membrane, a polyethersulfone membrane, a nylon membrane, a glass fiber, a nonwoven fabric, and a fabric. Among these, a nitrocellulose membrane is preferable from the viewpoint that this tends to make it easier to fabricate the labeled body-containing section, and the capturing body-immobilized section and the substrate zone described below. Note that the moving speed of the sample developed on the matrix can be changed by changing the material, the size, the pore size, the distribution, and the like of the porous membrane, and can be adjusted as appropriate depending on the type and the concentration of the test substance, the purpose of detection, and the like.

The size of the matrix is not particularly limited, but the matrix may be, for example, in a strip (elongated piece) shape having a width of about 3 to 10 mm and a length of about 30 to 100 mm. The thickness of the matrix is also not particularly limited, but may be, for example, within a range of 100 µm to 1 mm.

### [Labeled Body-containing Section]

The labeled body-containing section according to the present invention functions as a labeled body-holding section that elutably holds the blocked labeled antibody (hereinafter, sometimes referred to simply as the "labeled body"). The labeled body-containing section may be integrated with the matrix 2 as the labeled body-containing section 4 as shown in FIGs. 1 to 2, or may be such that the labeled body is elutably held relative to a separate water-absorbing material as a labeled body-containing pad 4a, and this is laminated on a labeled body zone 4b of the matrix 2 as shown in FIG. 3. In this case, the labeled body-containing pad 4a may also be caused to function as a sample supply section that receives a sample dropped or applied. The water-absorbing material is preferably a porous material, and includes, for example, the above porous membrane.

The size of the labeled body-holding section is not particularly limited, but may be, for example, within ranges of a width of 1 to 10 mm and a length of 3 to 30 mm. The thickness of the labeled body-containing pad may be, for example, within a range of 0.3 to 2 mm.

The method for holding the labeled body on the labeled body-containing section according to the present invention is not particularly limited as long as the method enables the labeled body to be eluted onto a sample (preferably, an aqueous solution or a water dispersion liquid) or a developer described below when the sample or the developer is developed, and includes, for example, a method that applies a solution containing the labeled body to the material of the matrix or the labeled body-containing pad or impregnates the material with the solution, followed by drying. The solution which is caused to contain the labeled body includes, for example, a buffer solution. The buffer solution includes a buffer solution adjustable to pH favorable for the target antigen-antibody reaction, and more specifically, a phosphate buffer solution, a Tris buffer solution, a Good's buffer solution, a boric acid buffer solution, and the like. The amount of the labeled body to be held on the labeled body-containing section according to the present invention may be adjusted as appropriate depending on the form of the sample, the sensitivity of the antibody contained in the labeled body to the test substance, the purpose of detection, and the like.

In the immunochromatographic strip of the present invention, once the sample comes into contact with the labeled body-containing section, the labeled body is eluted, and in the case where the test substance exists in the sample, a complex (an immune complex) in which the test substance is bound to the labeled body by antigen-antibody reaction between the test substance and the antibody contained in the labeled body is formed.

### [Capturing Body-immobilized Section]

In addition, in the immunochromatographic strip of the present invention, it is preferable that the matrix further comprise a capturing body-immobilized section (for example, a capturing body- immobilized section 6 shown in FIG. 1 and FIG. 2) to which a capturing body capable of capturing a test substance bound to the blocked labeled antibody is immobilized.

The capturing body-immobilized section according to the present invention functions as a detection zone of the immunochromatographic strip of the present invention. The capturing body-immobilized section according to the present invention is arranged downstream of the labeled body-containing section.

In the present invention, the "capturing body capable of capturing a test substance bound to the blocked labeled antibody (hereinafter, sometimes referred to simply as the "capturing body")" is a substance capable of binding to a test substance bound to the labeled body and capable of capturing the labeled body by means of the test substance.

Such a capturing body is typically an antibody capable of specifically binding to the test substance or an antibody capable of specifically binding to a binding portion between the test substance and an antibody contained in the labeled body. Although the antibody contained in such a capturing body may be the same as or different from the antibody contained in the labeled body, it is preferable that the antibodies do not compete in binding to the test substance depending on the recognized portion of the test substance.

Note that although the capturing body-immobilized section 6 is formed in a line shape in FIG. 1 and the like, the shape of the capturing body-immobilized section according to the present invention is not limited to this, and may be formed into any desired shape such as a circular shape or a polygonal shape. The shape of the capturing body-immobilized section according to the present invention is preferably a line shape, and more preferably a line shape having a width of 0.5 to 3.0 mm, for example.

In addition, the capturing body-immobilized section according to the present invention may be a plurality of capturing body-immobilized sections (two capturing body-immobilized sections 6a and 6b in FIGs. 3 to 4) depending on the type of the test substance and the detection method as shown in FIGs. 3 to 4. For example, in the case where a labeled body A containing an antibody a capable of binding to a test substance a and a labeled body B containing an antibody b capable of binding to a test substance b are used as labeled bodies, a capturing body-immobilized section A on which the antibody capable of binding to the test substance a is immobilized and a capturing body-immobilized section B on which the antibody capable of binding to the test substance b is immobilized may be correspondingly included.

The method for immobilizing the capturing body on the capturing body-immobilized section according to the present invention is not particularly limited, and a conventionally publicly-known method or a method based on this may be employed, and such a method includes, for example, a physical adsorption method and a method that binds the capturing body to the matrix using covalent bonding, and blocking may be conducted as necessary. The amount of the capturing body to be immobilized on the capturing body-immobilized section according to the present invention may be adjusted as appropriate depending on the form of the sample, the type of the antibody contained in the labeled body, the purpose of detection, and the like.

In the immunochromatographic strip of the present invention, once the labeled body which has bound to the test substance and has formed a complex moves to the capturing body-immobilized section, the complex is captured by the capturing body immobilized on the capturing body-immobilized section by means of the test substance, and the labeling substance forming the complex accumulates in the capturing body-immobilized section. As a result, since the capturing body-immobilized section exhibits a signal due to the accumulation of the labeling substance, this enables detection of a test substance in the sample by detecting this signal.

### [Development Confirmation Zone]

Moreover, in the immunochromatographic strip of the present invention, the matrix may further comprise a development confirmation zone (for example, a development confirmation zone 10 shown in FIG. 3 and FIG. 4) on which a substance capable of capturing the labeled body is immobilized.

The development confirmation zone according to the present invention functions as a control zone at which whether or not development of the developer has been successful in the immunochromatographic strip of the present invention to determine whether or not the test has been established. The development confirmation zone according to the present invention is arranged downstream of the labeled body-containing section. In addition, it is preferable that the development confirmation zone according to the present invention be arranged downstream of the capturing body-immobilized section.

In the present invention, the "substance capable of capturing the blocked labeled antibody (labeled body) (hereinafter, sometimes referred to as the "control body")" is a substance capable of binding to the labeled body and capable of capturing the labeled body.

Such a control body is preferably a substance that does not bind to a test substance, and is typically an antibody capable of binding to the labeling substance or the antibody contained in the labeled body. As such an antibody, for example, in the case where the labeling substance contained in the labeled body is an enzyme, an antienzyme may be used, and in the case where the antibody contained in the labeled body is a rabbit polyclonal antibody specific to the test substance, an anti-rabbit immunoglobulin antibody may be used.

Note that although the development confirmation zone 10 is formed in a line shape in FIG. 4, the shape of the development confirmation zone according to the present invention is not limited to this, and may be formed into any desired shape such as a circular shape or a polygonal shape. Among these shapes, the shape of the development confirmation zone according to the present invention is preferably a line shape, and more preferably a line shape having a width of 0.5 to 3.0 mm, for example.

The method for immobilizing the control body on the development confirmation zone according to the present invention is not particularly limited, and a conventionally publicly-known method or a method based on this may be employed, and such a method includes the same methods as those given as the above method for immobilizing the capturing body on the capturing body-immobilized section. The amount of the control body to be immobilized on the development confirmation zone according to the present invention may be adjusted as appropriate depending on the form of the sample, the types of the labeling substance and the antibody contained in the labeled body, the purpose of detection, and the like.

In the immunochromatographic strip of the present invention, once the labeled body (the labeled body that has not been captured by the capturing body-immobilized section in the case where the development confirmation zone is arranged downstream of the capturing body-immobilized section) moves to the development confirmation zone, the labeled body is captured by the control body immobilized on the development confirmation zone, so that the labeling substance contained in the labeled body accumulates in the development confirmation zone. As a result, since the development confirmation zone exhibits a signal due to the accumulation of the labeling substance, it is possible to confirm that the labeled body has been developed to the development confirmation zone by detecting this signal.

### [Absorption Zone]

In the immunochromatographic strip of the present invention, the matrix may further comprise an absorbent zone (for example, an absorbent zone 5 shown in FIGs. 1 to 3 and FIG. 5) as a member having a function of absorbing the sample and the developer that have moved on the matrix, which is a chromatographic medium, the labeled body that has not been captured by the capturing body-immobilized section or the development confirmation zone, and the like. The absorbent zone according to the present invention is preferably arranged on the most downstream side of the matrix.

The absorbent zone may be integrated with the matrix 2, but is preferably a member (absorbent pad) made of a separate water-absorbing material like the absorbent zone 5. The material of the absorbent zone is not particularly limited, and includes, for example, water-absorbing materials such as a cellulose paper filter, a nonwoven fabric, a fabric, and a cellulose acetate membrane. In addition, the absorbing material may contain silicon-containing particles or the like.

The size of the absorbent zone is not particularly limited, but may be, for example, within ranges of a width of 1 to 10 mm and a length of 4 to 6 mm, and the thickness of the absorbent pad may be, for example, within a range of 0.5 to 2 mm.

### [Substrate Zone]

In the immunochromatographic strip of the present invention, in the case where the labeling substance contained in the labeled body is an enzyme, the matrix may further comprise a substrate zone (for example, a substrate zone 7 shown in FIG. 3) that elutably holds the substrate of the enzyme. The substrate of the enzyme may be used by being added to the developer but may be held on the immunochromatographic strip in advance.

The method for holding the substrate on the substrate zone according to the present invention is not particularly limited as long as the method enables the substrate to be eluted onto the sample or the developer when the sample or the developer is developed, and includes the same method as those given as the above method for holding the labeled body on the labeled body-containing section. The amount of the substrate to be held on the substrate zone according to the present invention may be adjusted as appropriate depending on the types of the enzyme and the substrate, and the like.

In addition, as shown in FIG. 3 and FIG. 5, the immunochromatographic strip of the present invention may further comprise a developer pad (a developer pad 3 in FIG. 3 and FIG. 5) for supplying the developer to the matrix, and may further comprise a sheet (not shown, for example, an adhesive sheet made of a plastic, a metal, paper, or the like) for supporting the matrix and each pad, as necessary.

An immunochromatographic device of the present invention only has to comprise the immunochromatographic strip of the present invention. The form of the immunochromatographic device is not particularly limited, and, for example, the immunochromatographic device may further comprise a developer tank (a developer tank 11 in FIG. 3 and FIG. 5) for storing the developer, a protruding portion (a protruding portion 13 in FIG. 5) for supplying the developer in the developer tank to the matrix trough the developer pad, a pressing portion (a pressing portion 12 in FIG. 3 and FIG. 5) for pressuring and moving the protruding portion, and the like, upstream of the matrix of the immunochromatographic strip.

### (Method for Detecting test substance)

A method for detecting test substance of the present invention is a method, in the immunochromatographic strip of the present invention, comprising:
a labeling step of bringing a sample and the blocked labeled antibody into contact to form a complex of a test substance in the sample and the blocked labeled antibody;
a capturing step of capturing the complex; and
a detecting step of detecting the captured complex.

Hereinafter, one embodiment of the method for detecting test substance of the present invention will be described by giving a method for detecting test substance (an immunochromatography method) using the immunochromatographic strip in FIG. 1 or FIG. 3 as an example.

First, the sample is dropped on or applied to the matrix 2 such that the matrix 2 receives the sample (Step 1). The sample is preferably received such that the sample comes into direct contact with the labeled body, and for example, the sample 9 is preferably dropped on or applied to a drop zone 8 on the labeled body-containing section 4 (labeled body pad 4a) as shown in FIG. 3.

In addition, the developer may be dropped on, instilled into, or applied to the matrix 2 at the same position as or upstream of the labeled body-containing section 4 simultaneously or separately with the sample. The developer includes, for example, a buffer solution, and the buffer solution includes a buffer solution adjustable to pH favorable for the target antigen-antibody reaction, and more specifically, a phosphate buffer solution, a Tris buffer solution, a Good's buffer solution, a boric acid buffer solution, a 2-amino-2-methyl-1-propanol (AMP) buffer solution, and the like.

Moreover, in the case where the labeling substance contained in the labeled body is an enzyme, a substrate solution may be dropped on or applied to the matrix 2 simultaneously or separately with the sample or the developer. The solvent of the substrate includes, for example, the above buffer solutions.

The sample received in the matrix 2 moves to the labeled body-containing section 4 under capillary action. In this way, the sample is brought into contact with the labeled body contained in the labeled body-containing section 4 to elute the labeled body, and in the case where the test substance exists in the sample, a complex (an immune complex) of the test substance and the labeled body is formed by antigen-antibody reaction between the test substance and the antibody contained in the labeled body (Step 2, the labeling step).

In the case where the immunochromatographic strip of the present invention is the aspects shown in FIG. 3 to FIG. 5, for example, the sample 9 dropped on the drop zone 8 elutes the labeled body from the labeled body pad 4a, and is received in the matrix 2 in the labeled body zone 4b. At this time, in the case where the test substance exists in the sample, a complex of the test substance and the labeled body is formed. The sample and the labeled body received in the matrix 2 are developed in the matrix 2 under capillary action. In addition, by pressurizing the pressing portion 12 to move the protruding portion 13, it is possible to insert and immerse the developer pad 3 in the developer tank 11, and supply the developer to the matrix 2 through the developer pad 3. In this case, when the developer passes through the substrate zone 7, the substrate is eluted into the developer, and the developer containing the substrate flows. In the matrix 2, the sample and the labeled body (or the complex) are also eluted into the developer, and the developer containing the substrate, the labeled body (or the complex), and the sample flows.

Subsequently, the complex moves to the downstream side under capillary action to reach the capturing body-immobilized section 6 (detection zone 6). In this way, the complex is captured by the capturing body immobilized on the capturing body-immobilized section 6 by means of the test substance in the capturing body-immobilized section 6 (Step 3, the capturing step) . As a result of the complex being captured in the capturing body-immobilized section 6, the capturing body-immobilized section 6 exhibits color reaction or the like due to the labeling substance contained in the labeled body, which can be detected as a signal (the detecting step), and can be quantified as necessary.

In the method for detecting test substance of the present invention, the capturing step and the detecting step may be simultaneously conducted, or the detecting step may be conducted after the capturing step. For example, after the complex is captured in the capturing step, the detecting step of conducting processing (addition of the substrate, observation with a fluorescence microscope, detection with a fluorometer, detection with a scintillation counter, or the like) depending on the labeling substance contained in the complex may be executed.

In the case where the test substance is not contained in the sample, since the labeled body is not immobilized on the capturing body-immobilized section 6 and moves further downward, the signal is not detected in the capturing body-immobilized section 6. In addition, in the case where the concentration of the test substance in the sample is low, since the amount of the labeling substance accumulated in the capturing body-immobilized section 6 becomes low, a sufficient signal tends to be not detected in general (the signal intensity is weak). However, according to the detection method using the immunochromatographic strip of the present invention, since the signal intensity becomes sufficiently high even when the concentration of the test substance is low, this enables detection of a test substance with high sensitivity.

In addition, the labeled body eluted from the labeled body-containing section 4 (the labeled body that has not captured in the capturing body-immobilized section 6 in the case where the development confirmation zone 10 is arranged downstream of the capturing body-immobilized section 6) moves to the downstream side under capillary action to reach the development confirmation zone 10 as well. In this way, in the development confirmation zone 10, the labeled body is captured and detected by the control body immobilized on the development confirmation zone 10 (Step 4). Note that in the immunochromatographic strip 101 of FIG. 1, Step 4 is conducted after Step 3, but Step 3 and Step 4 may be independent from each other or may be simultaneously conducted at the same position, or Step 3 may be conducted after Step 4. As a result of the labeled body being captured in the development confirmation zone 10, the development confirmation zone 10 exhibits color reaction or the like due to the labeling substance contained in the labeled body, which can be detected as a signal and may be quantified as necessary to determine that the sample has moved with no trouble on the immunochromatographic strip. The criterion for the determination may be set as appropriate depending on the purpose of the detection, the type of the test substance, and the like. The developer is eventually absorbed by the absorbent zone 5 located on the downstream side.

### <Immunochromatographic Kit>

An immunochromatographic kit of the present invention comprises at least the immunochromatographic strip of the present invention. The immunochromatographic strip may be included as the immunochromatographic device of the present invention.

In addition, the immunochromatographic kit of the present invention may further comprise at least one selected from the group consisting of a diluent of a sample, a pretreatment liquid, and the developer as necessary. Moreover, the labeled body, the capturing body, the control body, and the like may be combined. In addition, for example, in the case where the labeling substance is an enzyme, a substrate necessary for detecting a signal, a reaction stop solution, and the like may be included. In addition, the immunochromatographic kit of the present invention may also include an instruction manual for the kit.

The immunochromatographic strip, the immunochromatographic device, the method for detecting test substance, and the immunochromatographic kit of the present invention may be used not only for research but also, as a pharmaceutical product for in-vitro diagnosis, for the detection of a test substance based on which diseases related to the test substance are diagnosed, for example.

### [Examples]

The present invention will be described more specifically based on Examples and Comparative Examples; however, the present invention is not limited to Examples below. Particularly, the type of each buffer, reaction conditions, purification conditions, and the like may be adjusted as appropriate depending on the types and amounts of an antibody and a polymer.

### (1) Preparation of Blocked Labeled Antibody (Blocked Labeled Anti-HIV Antibody)

### 1. Preparation of Antibody Digestion (Antibody Fragment (F(ab)₂))

First, a buffer of an anti-HIV lp24 monoclonal antibody (mouse, IgG, anti-HIV antibody (hiv)) was substituted with a 0.1M citric acid buffer (pH 3.5). Subsequently, 2.5 mg/mL pepsin was added such that IgG:pepsin=100:1 (w:w), followed by reacting at 37°C for 1 hour. Then, 2M Tris-HCl buffer (pH 10.0) in 1/10 (v/v) relative to the reaction liquid after the reaction was added and the reaction was stopped. The obtained product was concentrated to an appropriate solution volume, followed by gel filtration purification to obtain an antibody fragment F(ab')₂ (F(hiv)). The gel filtration purification was conducted under conditions of the buffer : 1mM EDTA·2Na, 0.1M phosphoric acid buffer (pH 6.3); the device: AKTA purifier 10 (manufactured by GE Healthcare Life Sciences) ; the column: Superdex 200 Increase 10/300 GL (manufactured by GE Healthcare Life Sciences).

### 2. Preparation of Hydrazine dextran

First, 240 mg of 70 kDa or 250 kDa dextran (manufactured by CarboMer, (D)) was added to 4.8 mL of a 0.1M phosphoric acid buffer (pH 7.0), which was then agitated in the dark 25°C for 30 minutes to be dissolved. Subsequently, ion-exchange water and NaIO₄ (oxidant) were added such that the final concentration of NaIO₄ became 50 mm, 60 mm, or 65 mm, followed by agitating in the dark at 25°C for 30 minutes. Buffer exchange was conducted with 0.1M phosphoric acid buffer (pH 6.0) using PD-10 column (manufactured by GE Healthcare Life Sciences, column: Sephadex G-25.) to obtain 20 mL of a solution. To the solution, 5.04 g of NH₂NH₂·HCl was added, followed by agitating in the dark at 25°C for 2 hours. Then, 800 mg of DMAB(dimethylamine-borane) was added, followed by further agitating in the dark at 25°C for 2 hours. Dialysis with 4 L of ion-exchange water was conducted using RC50K (regenerated cellulose having a molecular weight of 50,000) dialysis membrane in the dark for 3 hours, and thereafter the obtained product was left to stand at 4°C overnight. Buffer exchange was conducted through gel filtration (column: Sephadex G-25) using a 0.1M phosphoric acid buffer (pH 6.0), followed by adjusting such that the concentration of dextran became 1.0 mg/mL to obtain each hydrazine dextran solution.

### 3. Preparation of Dextran-enzyme Conjugate (Label carrier, DA)

Buffer exchange was conducted through gel filtration (column: Sephadex G-25) using a 0.1M phosphoric acid buffer (pH 6.0) on 20.0 mL of alkaline phosphatase of 10 mg/mL (manufactured by Oriental Yeast Co., Ltd. "ALP-50", ALP, (A)) to prepare 63.4 mL of a solution of 3.0 mg/mL. Then, 31.7 mL of 27 mmNaIO₄ was added (final concentration of NaIO₄: 9 mm), followed by agitating in the dark at 25°C for 30 minutes. Buffer exchange was conducted through gel filtration (column: Sephadex G-25) using a 0.1M phosphoric acid buffer (pH 6.0) to obtain an aldehyde ALP solution of 0.5 mg/mL.

To this, each hydrazine dextran solution of 1.0 mg/mL obtained in the above 2. was added such that the concentration of the hydrazide group (amino group) became 25 µm, followed by agitating in the dark at 25°C for 16 hours. Subsequently, 47 mg of DMAB was added, followed by agitating in the dark at 25°C for 2 hours, and thereafter, 5.6 mL of 1.5M Tris-HCl buffer (pH 9.0) was added, followed by agitating in the dark at 25°C for 2 hours. An ultrafiltration module (Pellicon XL50, manufactured by Merck Millipore) was attached to Labscale TFF System (manufactured by Merck Millipore) to concentrate the solution to 15 mL, and buffer exchange was conducted through gel filtration (column: Sephadex 200pg) using a 0.1M phosphoric acid buffer (pH 7.0) to obtain 10.9 mL of each dextran-enzyme conjugate(DA) solution of 3.0 mg/mL.

### 4. Thiolation of Antibody

First, 0.2 mol/L 2-mercaptoethylamine (2MEA) in 1/20 (v/v) relative to the antibody after antibody digestion (pepsin digestion) obtained in the above 1. was added and reacted at 37°C for 1.5 hours. The buffer of the reaction liquid after the reaction was substituted with 1mM EDTA·2Na, 0.1M phosphoric acid buffer (pH 6.3).

### 5. Maleimidation of Dextran-enzyme conjugate

Sulfo-E MCS was added to the dextran-enzyme conjugate obtained in the above 3. such that ALP:Sulfo-E MCS=1:40 (molar ratio) to be reacted at 25°C for 1 hour. After the reaction, the buffer of the reaction liquid was substituted with 1mM EDTA·2Na, 0. 1M phosphoric acid buffer (pH 6.3).

### 6. Coupling (Preparation of Blocked Labeled Anti-HIV Antibody)

First, the antibody fragment after the thiolation obtained in the above 4. and the maleimide dextran-enzyme conjugate (DA) obtained in the above 5. were mixed such that 4.5:1 (molar ratio), and reacted at 4°C overnight (coupling). Subsequently, 0.2 mol/L 2MEA in 1/100 (v/v) relative to the reaction liquid was added and reacted at 25°C for 30 minutes. Subsequently, 0.2 mol/L iodoacetamide (IAA) in 1/50 (v/v) relative to the reaction liquid was added and reacted at 25°C for 30 minutes, and thereafter, gel filtration purification was conducted to obtain a blocked labeled anti-HIV antibody (DAF(hiv)) in which ALP(A) and anti-HIV antibody (F(hiv)) bonded to dextran (D). The gel filtration purification was conducted under condition that buffer: 0.1M Tris-HCl(pH 8.0); device: AKTA purifier 10 (manufactured by GE Healthcare Life Sciences); column: Superose 6 Increase 10/300 GL or Superose 6 10/300 GL (manufactured by GE Healthcare Life Sciences). Each fraction after the gel filtration purification was subjected to antigen-antibody reaction with HIV antigen (WHO international standard), and the fractions with which favorable reactivity was obtained were pooled and used for each test later.

### (Blocked Labeled Anti-influenza Antibody)

Antibody fragments F(ab')₂ (F(fluA) or F(fluB)) were obtained in the same manner as in the case of the blocked labeled anti-HIV antibody except that an anti-influenza A monoclonal antibody (IgG, (fluA)) or an anti-influenza B monoclonal antibody (IgG, (fluB)) was used instead of the anti-HIV 1p24 monoclonal antibody (IgG, (hiv)) in the antibody digestion step of the above 1.

Using each antibody fragment F(ab')₂ thus obtained, a blocked labeled anti-influenza antibody (DAF(fluA) or DAF(fluB)) in which ALP and the anti-influenza antibody A or B bonded to dextran was obtained in the same manner as in the case of the blocked labeled anti-HIV antibody except that the antibody fragment F(ab')₂ was mixed such that the ratio between the antibody fragment (F(fluA)) after the thiolation and the maleimide dextran-enzyme conjugate (DA) became 3:1 (molar ratio) or such that the ratio between the antibody fragment (F(fluB)) after the thiolation and the maleimide dextran-enzyme conjugate (DA) became 2:1 (molar ratio) in the coupling step of the above 6. For the blocked labeled anti-influenza antibodies thus obtained, each fraction after the gel filtration purification was subjected to antigen-antibody reaction with recombinant influenza A antigen (manufactured by FUJIREBIO Inc.) or recombinant influenza B antigen (manufactured by FUJIREBIO Inc.), and the fractions with which favorable reactivity was obtained were pooled and used for each test later.

### (2) Measurement of Average Particle Size

The average particle size of each dextran-enzyme conjugate (DA) in the 0.1M phosphoric acid buffer (pH 7.0) obtained in the dextran-enzyme conjugate preparation step of 3. in (1) was measured. The DA concentration of each DA solution was adjusted to be 0.07 to 0.8 mg/mL with the buffer, and the measurement and analysis were conducted using Zeta potential·particle size·molecular weight measurement system (ELSZ-2000ZS, manufactured by OTSUKA ELECTRONICS Co., LTD.) under conditions set in the system by default:

### [Measurement Conditions]

Sampling time: N/A (µs), The number of correlation channels: 475 (ch),
Correlation method: TD, Cumulated number: 25 (times),
Pinhole: 50 (µm),
Measurement position Z: 4.900 (mm), X: 5.850 (mm),
Cell type: Size Cell, Measurement angle: 165.0 (°),
Temperature: 25.0 (°C), Solvent name: WATER,
Refractive index of solvent: 1.3328, Viscosity of solvent: 0.8878 (cP),
Scattering intensity: 75585 (cps)
[Analysis conditions]
Analyzing method: CONTIN, Analysis range: 1.0-1400.0 (nm),
Cut Left: 0, Right: 0, Fitting range: 1.003-2,
Noise cut level: 1 (%), Residual error: 1.763e-003 [OK],
Scattering factor: RGD.
Results of the measurement are shown in Table 1 below together with the concentration of NaIO₄ (oxidant), the mass of dextran used, and the DA concentration of the DA solution used for the measurement in the hydrazine dextran preparation step of the above 2. in (1).

**[Table 1]**

| ID | Concentration of the oxidant [mM] | Mass of dextran [Da] | Concentration of DA [mg/mL] | Average particle size [nm] |
|---|---|---|---|---|
| A | 50 | 70k | 0.75 | 43.4 |
| B | 60 | 70k | 0.1 | 119.6 |
| C | 65 | 70k | 0.75 | 89.1 |
| D | 50 | 250k | 0.1 | 80.1 |

### (3) Preparation of Immunochromatographic Device

### (Example 1)

First, detection zones 6a and 6b as well as a development confirmation zone 10 were formed in a matrix 2 made of a nitrocellulose membrane (manufactured by Sartorius AG) having a width of 3.65 mm and a length of 50 mm shown in FIG. 3. In the formation of the detection zone 6a, 0.54 µL of an antigen solution containing 1.25 mg/mL of HIV antigen (antigen that does not bind to F (hiv) ) was spot-applied in a line shape at a position 16 mm from the end of the matrix 2 on the absorbent pad 5 side and was dried. In the formation of the detection zone 6b, 0.54 µL of an antibody solution (capturing body fluid) containing 2.0 mg/mL of anti-HIV-1p24 polyclonal antibody (rabbit, IgG) was spot-applied in a line shape at a position 13.3 mm from the end of the matrix 2 on the absorbent pad 5 side and was dried. In addition, in the formation of the development confirmation zone 10, 0.54 µL of a control body fluid containing 21.4 µg/mL of anti-ALP antibody was spot-applied in a line shape at a position 10.8 mm from the end of the matrix 2 on the absorbent pad 5 side and was dried. In addition, after the detection zones 6a and 6b as well as the development confirmation zone 10 are dried, blocking was conducted using 10.0 mg of a solution containing 0.1 mg/mL of alkali-treated casein.

Subsequently, 27.8 µg of BCIP (5-bromo-6-chloro-3-indolyl phosphate) was spot-applied in a line shape at a position 47 mm from the end of the matrix 2 on the absorbent pad 5 side and was dried to form a substrate zone 7.

Subsequently, 3.04 µL of a labeled body fluid containing 12.5 µg/mL of a blocked labeled anti-HIV antibody (DAF70A(hiv)) obtained using DA of ID: A in Table 1 in the above (1) and 21.2 µg/mL of ALP label HIV antigen was spot-applied to a porous sheet having a width of 3.65 mm and a length of 15.0 mm and was dried to form a labeled body pad 4a. The labeled body pad 4a thus obtained was arranged in a labeled body zone 4b between the detection zone 6a and the substrate zone 7 of the matrix 2 to obtain an immunochromatographic strip.

Subsequently, the immunochromatographic strip (the matrix 2, the labeled body pad 4a), the developer pad 3 (a glass fiber paper filter), and the absorbent pad 5 (a highly absorbent filter paper) laminated as shown in FIG. 3 were immobilized on a plastic case having a developer tank 11 and a pressing portion 12 as shown in FIGs. 4 to 5 to obtain an immunochromatographic device (an immunochromatographic device 102).

### (Example 2)

An immunochromatographic device was obtained in the same manner as in Example 1 except that a blocked labeled anti-HIV antibody (DAF70B(hiv)) obtained using DA of ID: B in Table 1 was used as the blocked labeled anti-HIV antibody in (1) instead of DAF70A(hiv).

### (Comparative Example 1)

A labeled anti-HIV antibody (AF(hiv)) in which ALP and anti-HIV antibody bonded was obtained in the same manner as in the blocked labeled anti-HIV antibody of (1) except that alkaline phosphatase (Oriental Yeast Co., Ltd.) was used instead of the dextran-enzyme conjugate (DA) in (1), GMBS was used instead of Sulfo-E MCS as a maleimidation reagent in the maleimidation step of 5. in (1), and gel filtration purification in the coupling step of 6. in (1) was conducted under the following conditions: the buffer: 1M ammonium sulfate in 0.1M phosphoric acid buffer (pH 7.0), 0.1M phosphoric acid buffer (pH 7.0); the device: AKTA purifier 10 (manufactured by GE Healthcare Life Sciences) ; the column: HiTrap Phenyl HP (manufactured by Healthcare Life Sciences) after hydrophobic chromatography was conducted. An immunochromatographic device was obtained in the same manner as in Example 1 except that the labeled anti-HIV antibody (AF(hiv)) thus obtained was used instead of the blocked labeled anti-HIV antibody.

### (Example 3)

First, detection zones 6a and 6b as well as a development confirmation zone 10 were formed in a matrix 2 made of a nitrocellulose membrane (manufactured by Sartorius AG) having a width of 3.65 mm and a length of 50 mm shown in FIG. 3. In the formation of the detection zone 6a, 0.54 µL of an antibody solution containing 1.0 mg/mL of anti-influenza A monoclonal antibody was spot-applied in a line shape at a position 16 mm from the end of the matrix 2 on the absorbent pad 5 side and was dried. In the formation of the detection zone 6b, 0.54 µL of an antibody solution containing 1.0 mg/mL of anti-influenza B monoclonal antibody was spot-applied in a line shape at a position 13.5 mm from the end of the matrix 2 on the absorbent pad 5 side. In addition, in the formation of the development confirmation zone 10, 0.54 µL of a control body fluid containing 57.1 µg/mL of anti-ALP antibody was spot-applied in a line shape at a position 11 mm from the end of the matrix 2 on the absorbent pad 5 side.

Subsequently, 73.6 µg of BCIP was spot-applied in a line shape at a position 47.5 mm from the end of the matrix 2 on the absorbent pad 5 side and was dried to form a substrate zone 7.

Subsequently, 3.04 µL of a labeled body fluid containing both 5.36 µg/mL of a blocked labeled anti-influenza A antibody (DAF250(fluA)) obtained using DA obtained in the same manner as DA of ID: D in Table 1 in the above (1) and 8.04 µg/mL of a blocked labeled anti-influenza B antibody (DAF250(fluB)) was spot-applied to a porous sheet having a width of 3.65 mm and a length of 15.0 mm and was dried to form a labeled body pad 4a. The labeled body pad 4a thus obtained was arranged in a labeled body zone 4b between the detection zone 6a and the substrate zone 7 of the matrix 2 to obtain an immunochromatographic strip.

Subsequently, the immunochromatographic strip (the matrix 2, the labeled body pad 4a), the developer pad 3 (a glass fiber paper filter), and the absorbent pad 5 (a highly absorbent filter paper) laminated as shown in FIG. 3 were immobilized on a plastic case having a developer tank 11 and a pressing portion 12 as shown in FIGs. 4 to 5 to obtain an immunochromatographic device (the immunochromatographic device 102).

### (Comparative Example 2)

A labeled anti-influenza A antibody (AF(fluA)) and a labeled anti-influenza B antibody (AF(fluB)) in each of which ALP and an anti-influenza antibody bonded were obtained in the same manner as in the blocked labeled anti - influenza antibody of (1) except that an amount ratio between the anti-influenza A monoclonal antibody or anti-influenza B monoclonal antibody (IgG) and pepsin (IgG:pepsin) was changed to 200:1 (w:w) in the antibody digestion of 1. in (1), alkaline phosphatase (Oriental Yeast Co., Ltd.) was used instead of the dextran-enzyme conjugate (DA) of (1), GMBS was used instead of Sulfo-E MCS as a maleimidation reagent in the maleimidation step of 5. in (1), and the antibody fragment and the ALP were mixed such that the ratio between the antibody fragment (F(fluA)) after the thiolation and ALP became 2:1 (molar ratio) or such that the ratio between the antibody fragment (F(fluB)) after the thiolation and ALP became 3:1 (molar ratio) in the coupling step of 6. in (1). An immunochromatographic device was obtained in the same manner as in Example 3 except that the labeled anti-influenza A antibody (AF(fluA)) and the labeled anti-influenza B antibody (AF(fluB)) thus obtained were used instead of the blocked labeled anti-influenza A antibody or the blocked labeled anti-influenza B antibody, and a nitrocellulose membrane manufactured by Merck KGaA was used as the nitrocellulose membrane.

### (4) Test substance Detection Test (Immunochromatographic Test)

### (Examples 1 to 2, Comparative Example 1)

HIV-1p24 antigen (WHO international standard) was diluted with a negative sample (manufactured ProMedDx, LLC) to 8.0, 4.0, 2.0, 1.6, 1.0, or 0.8 IU/mL to prepare each sample. Then, 25 µL of each sample was dropped on the drop zone 8 of the immunochromatographic device prepared in Example 1, 2, or Comparative Example 1 of (3) . Subsequently, the pressing portion 12 was pressed down to develop the developer, and the color production of the detection zones 6a and 6b as well as the development confirmation zone 10 15 minutes after the pressing was visually observed. The determination was made based on the following criteria:
Positive: A blue line was observed in the detection zone 6b and the development confirmation zone 10
Negative: A blue line was observed in the development confirmation zone 10, but no color production was observed in the detection zone 6a or the detection zone 6b. Note that the color production in the development confirmation zone 10 was observed in all of the immunochromatographic devices. The test was conducted on two (Lots. 1 to 2) of each immunochromatographic device. The results are shown in Table 2 below.

**[Table 2]**

| HIV-1p24 antigen [IU/mL] | Lot. | Comparative Example 1 | Example 1 | Example 2 |
|---|---|---|---|---|
| | | AF | DAF70A | DAF70B |
| 8.0 | 1 | Positive | Positive | Positive |
| | 2 | Positive | Positive | Positive |
| 4.0 | 1 | Negative | Positive | Positive |
| | 2 | Negative | Positive | Positive |
| 2.0 | 1 | Negative | Positive | Positive |
| | 2 | Negative | Positive | Positive |
| 1.6 | 1 | Negative | Positive | Positive |
| | 2 | Negative | Positive | Positive |
| 1.0 | 1 | Negative | Negative | Positive |
| | 2 | Negative | Negative | Positive |
| 0.8 | 1 | Negative | Negative | Negative |
| | 2 | Negative | Negative | Negative |

### (Example 3, Comparative Example 2)

The recombinant influenza A antigen (concentration: 625 pg/mL) or the recombinant influenza B antigen (concentration: 8.75 ng/mL) was diluted into 2-, 4-, 8-, 16-, 32-, 48-, 64-, or 96-fold with a sample treatment solution to prepare each sample. Then, 20 µL of each sample was dropped on the drop zone 8 of the immunochromatographic device prepared in Example 3 or Comparative Example 2 of (3). Subsequently, the pressing portion 12 was pressed down to develop the developer, and the color production of the detection zones 6a and 6b as well as the development confirmation zone 10 15 minutes after the pressing was visually observed. The determination was made based on the following criteria:
in the case where the sample contained the recombinant influenza A antigen,
   Positive: A blue line was observed in the detection zone 6a and the development confirmation zone 10
   Negative: A blue line was observed in the development confirmation zone 10, but no color production was observed in the detection zone 6a or the detection zone 6b,
in the case where the sample contained the recombinant influenza B antigen,
   Positive: A blue line was observed in the detection zone 6b and the development confirmation zone 10
   Negative: A blue line was observed in the development confirmation zone 10, but no color production was observed in the detection zone 6a or the detection zone 6b.

Note that the color production in the development confirmation zone 10 was observed in all of the immunochromatographic devices. The results are shown in Table 3 below.

**[Table 3]**

| Recombinant influenza A antigen | | |
|---|---|---|
| Dilution factor | Example 3 | Comparative Example 2 |
| | DAF250 | AF |
| 4 | Positive | Positive |
| 8 | Positive | Negative |
| 16 | Positive | Negative |
| 32 | Positive | Negative |
| 64 | Positive | Negative |
| 96 | Negative | - |

| Recombinant influenza B antigen | | |
|---|---|---|
| Dilution factor | Example 3 | Comparative Example 2 |
| | DAF250 | AF |
| 2 | Positive | Positive |
| 4 | Positive | Positive |
| 8 | Positive | Negative |
| 16 | Positive | Negative |
| 32 | Negative | Negative |
| 48 | Negative | Negative |

As shown in Table 2 and Table 3, it was confirmed that by using the immunochromatographic device (for example, Example 1 to 3) comprising the immunochromatographic strip containing the blocked labeled antibody according to the present invention, it was possible to detect a test substance with high sensitivity even for a sample in which the concentration of the test substance is low as compared with the immunochromatographic device (for example, Comparative Examples 1 to 2) comprising the immunochromatographic strip containing the labeled antibody that contain no polymer carrier.

### [Industrial Applicability]

As described above, the present invention makes it possible to provide an immunochromatographic strip that is capable of detection of a test substance with high sensitivity even when the concentration of the test substance is low, an immunochromatographic device comprising the same, an immunochromatographic kit comprising at least one of these, and a method for detecting test substance that is capable of detection of a test substance with high sensitivity by immunochromatography even when the concentration of the test substance is low. The test substance includes, for example, viruses that cause infectious diseases. Since it is possible to detect a virus with higher sensitivity than the conventional techniques even in an initial stage of an infection when the concentration of the virus in the body is low, the present invention not only can be used in research but also can greatly contribute to a diagnosis and the like, particularly an early diagnosis of a disease caused by the virus.

### [Reference Signs List]

101 ... immunochromatographic strip, 102 ... immunochromatographic device, 2 ... matrix, 3 ... developer pad, 4, 4b, 4a ... labeled body-containing section (4a ... labeled body pad, 4b ... labeled body zone), 5 ... absorbent zone, absorbent pad, 6, 6a, 6b ... capturing body-immobilized section, detection zone, 7 ... substrate zone, 8 ... drop zone, 9 ... sample, 10 ... development confirmation zone, 11 ... developer tank, 12 ... pressing portion, 13 ... protruding portion, 14 ... direction of development

## Claims

1. An immunochromatographic strip for use in detecting a test substance in a sample by immunochromatography, comprising:
a labeled body-containing section containing a blocked labeled antibody in which a labeling substance and an antibody capable of binding to a test substance are immobilized on a water-soluble carrier made of a water-soluble polymer.

2. The immunochromatographic strip according to claim 1, wherein
an average particle size of the blocked labeled antibody is 50 nm or more.

3. The immunochromatographic strip according to claim 1 or 2, wherein
the water-soluble polymer is at least one selected from the group consisting of sugars and amino acids.

4. The immunochromatographic strip according to any one of claims 1 to 3, further comprising:
a capturing body-immobilized section on which a capturing body capable of capturing a test substance bound to the blocked labeled antibody is immobilized.

5. An immunochromatographic device for use in detecting a test substance in a sample by immunochromatography, comprising:
the immunochromatographic strip according to any one of claims 1 to 4.

6. An immunochromatographic kit for use in detecting a test substance in a sample by immunochromatography, comprising:
at least one selected from the group consisting of the immunochromatographic strip according to any one of claims 1 to 4 and the immunochromatographic device according to claim 5.

7. A method for detecting test substance for detecting a test substance in a sample by immunochromatography, comprising:
in an immunochromatographic strip comprising a labeled body-containing section containing a blocked labeled antibody in which a labeling substance and an antibody capable of binding to a test substance are immobilized on a water-soluble carrier made of a water-soluble polymer,
a labeling step of bringing a sample and the blocked labeled antibody into contact to form a complex of a test substance in the sample and the blocked labeled antibody;
a capturing step of capturing the complex; and
a detecting step of detecting the captured complex.

8. The method for detecting test substance according to claim 7, wherein
an average particle size of the blocked labeled antibody is 50 nm or more.

9. The method for detecting test substance according to claim 7 or 8, wherein
the water-soluble polymer is at least one selected from the group consisting of sugars and amino acids.

10. The method for detecting test substance according to any one of claims 7 to 9, wherein
the immunochromatographic strip further comprises a capturing body-immobilized section on which a capturing body capable of capturing a test substance bound to the blocked labeled antibody is immobilized, and
the capturing step is a step of capturing the complex by means of a test substance bound to the blocked labeled antibody in the capturing body-immobilized section.
